# EUROPEAN PATENT SPECIFICATION

(11) **EP 3 206 838 B1**
(45) Date of publication and mention of the grant of the patent: **19.12.2018**
(21) Application number: 15787490.0
(22) Date of filing: 13.10.2015
(51) Int. Cl.: A61B 17/16, A61B 17/88, B25B 21/00, B25B 23/14, A61B 90/00

(54) **COMBINATION SCREWDRIVER AND TORQUELIMITING SYSTEM**
KOMBINATIONSSCHRAUBENDREHER UND DREHMOMENTBEGRENZUNGSSYSTEM
TOURNEVIS À USAGES MULTIPLES ET SYSTÈME DE LIMITATION DE COUPLE

(30) Priority: 13.10.2014 NL 2013623
(43) Date of publication of application: 23.08.2017
(73) Proprietor: Mercator Innovations BVBA, 2970 Schilde (BE); Innortho BVBA, 3500 Hasselt (BE)
(72) Inventor: DIERICKX, Carl, B-3500 Hasselt (BE); FORTEMS, Yves, B-2970 Schilde (BE); WENMAKERS, Dirk, B-3650 Dilsen (BE)
(74) Representative: LC Patents
(86) International application number: PCT/EP2015/073655
(87) International publication number: WO 2016/059039

(56) References cited:
- DE-A1- 2 822 372
- DE-U1-202010 011 308

## Description

### FIELD OF THE INVENTION

The present invention relates to the field of combination rotary tools, more in particular in the combination of screwdrivers and drills. In particular, the invention relates to a sleeve member having a hollow portion adapted to embrace a drill, and comprising a bolt- or screw-fitting unit. More in particular, in the context of the present invention said sleeve member is provided with a built-in mechanism for torque limitation.

### BACKGROUND TO THE INVENTION

In certain types of surgery and construction works, it is necessary for the operator (surgeon or workman) to drill several holes and subsequently drive fastening screws or bolts into said holes. With conventional devices, the operator needs 2 separate devices, a drill and a separate screwdriver; or when using a single device, the operator, after drilling a hole, needs to change the drill ending on the motor unit with a screwdriver ending before he can fasten a screw or bolt. As evident, this changing of the ends takes time, and can distort the surgical or work flow.

As a solution for this problem, the present invention provides a hollow sleeve with a screw-or bolt-fitting socket/unit on one ending, which is adapted to embrace a drill. This type of slide-on screwdrivers is known for example from DE202010011308, DE2822372, US4218795, US5313680, US3932904, US3484114, EP2345496, US5409333 and US4796319. However, none of these addresses the aspects of torque limitation, let it be to provide a solution to the problems associated therewith.

In particular during surgery and often also in construction works, it is highly important to use a torque limiter to avoid damage to the device or damage to the areas of application of the screws or bolts and possible subsequent 'cold welding'. Evidently, each type/size of screw or bolt requires a suitably adapted torque limiter. Hence, on top of the changing process from drill to screwdriver, either a pre-installed torque limiter needs to be adjusted or an adjusted and regularly calibrated torque limiter needs to be installed in accordance with the type/size of screw or bolt. This process again takes precious time. Furthermore, when erroneously installing the wrong or non-calibrated torque limiter or erroneously adjusting the pre-installed torque limiter, in accordance with a certain screw or bolt, crucial errors will occur, especially during stressful situations such as urgent surgical procedures.

The present invention provides a solution to these problems by providing a hollow sleeve member with a screw-or bolt-fitting socket/unit on one ending, which is adapted to embrace a drill, and which is provided with a built-in mechanism for torque limitation. In a particular embodiment, said mechanism for torque limitation is adapted, depending on the type of bolt-or screw-fitting unit in said sleeve member. Hence, after drilling a hole, the operator can easily slide the screwdriver part (hollow sleeve) over the drill, and thus quickly switch from drilling to screwdriving, with the correct torque limiter readily available. Although, numerous torque limiting devices are known in the art, these are either integrated in the motor unit (US20110184425, WO2013178932) or provided as a separate unit to be installed (e.g. US3167936, US3942337, US6132435, EP1996371, EP2311397), however, these are not integrated in the screwdriver part, let it be pre-adjusted depending on the bolt- or screw-fitting unit at the end of the screwdriver part.

### SUMMARY OF THE INVENTION

In a first aspect, the present invention provides a sleeve member (1) comprising:
- a hollow portion (2) adapted to embrace a drill (13),
- a bolt- or screw-fitting unit (3) on a first end (1a) of said sleeve member (1), and
- attachment means (4) on a second end (1b) of said sleeve member (1), for releasably engaging said sleeve member (1) to said drill (13) or to a motor unit (14);
said sleeve member (1) being characterized in that it comprises a built-in mechanism for torque limitation (5).

In a particular embodiment, said built-in mechanism for torque limitation (5) is adapted, depending on the type of bolt- or screw-fitting unit (3) in said sleeve member (1).

In another particular embodiment, said built-in mechanism for torque limitation (5) is defined by one or more breakpoints (5a) in said sleeve member (1). Hence, said sleeve member is preferably for single use. In said particular embodiment, the resistance of said breakpoints (5a) is preferably adapted depending on the type of bolt- or screw-fitting unit (3) in said sleeve member (1). In this particular embodiment, the sleeve member (1) may be made from any suitable material, however, preferably from metal or plastic, most preferably from a plastic material, such as for example polyethylene (PE), polypropylene (PP), polystyrene (PS), polyether ether ketone (PEEK),...

In yet another particular embodiment, said built-in mechanism for torque limitation (5) is defined by one or more detents (5b), preferably at the second end (1b) of said sleeve member (1), said detents (5b) being adapted to receive one or more spring-rolling elements (8) from a torque limiter (11 or 19) mounted on said drill (13) or motor unit (14). In said particular embodiment, the size and/or form of said detents (5b) in said sleeve member (1) is preferably adapted, depending on the type of bolt- or screw-fitting unit (3). Furthermore, said detents (5b) may be asymmetric in shape thereby allowing to apply a different torque limitation in clockwise or counterclockwise direction. In this particular embodiment, the sleeve member (1) may be made from any suitable material such as for example a plastic material, such as polyethylene (PE), polypropylene (PP), polystyrene (PS), polyether ether ketone (PEEK) and the like; or from metal such as for example stainless steel.

In a further aspect the present invention provides a kit comprising a sleeve member (1) according to the present invention, and one or more bolts or screws adapted to fit the bolt-or screw fitting unit (3) on said first end (1a) of said sleeve member. These screws can preferably also be prefitted on the bolt-or screw fitting unit (3).

In yet a further aspect, the present invention provides a torque limiter (**11**) according to claim 9.

The torque limiter according to this invention, is capable of embracing any suitable type of driven member (12), such as a screwdriver, drill, ... In the context of the present invention, the driven member is preferably a drill (13). Hence, and in correspondence with the figures herein after, in the remainder of the description, the specific use of a drill (13) is included, with the understanding that it can be interchanged with any suitable type of driven member (12).

In yet a further aspect, the present invention provides a torque limiter (**19**), according to claim 10.

In a particular embodiment, the invention provides a torque limiter as defined herein above, wherein said second torque limiter element (7) comprises a groove to receive a first part (8a) of said one or more spring-rolling elements (8).

In a further embodiment, the present invention provides a combination of a torque limiter according to this invention, with a sleeve member (1) comprising a hollow portion (2) to embrace a drill (13), and releasably engages said second torque limiter element (7) to said drill (13) by means of said one or more spring-rolling elements (8) with the built-in mechanism for torque limitation (5) present on said sleeve member (1) in an indirect way. In said particular embodiment, the built-in mechanism for torque limitation (5) present on said sleeve member (1) preferably comprises one or more detents (5b) adapted to receive a second part of said spring-rolling elements (8b).

In yet a further embodiment, said one or more spring-rolling elements (8) each comprise 2 transmission balls (9) connected by an elastic element (10) such as for example one or more spring washer (10a) or a coil spring.

In yet a further embodiment, said driven element (12) may be a drill (13) or a screwdriver, preferably a drill, more in particular a drill which may be embraced by a hollow sleeve (the sleeve member (1)), which serves the purpose of a screwdriving element.

In yet a further embodiment, all components of the torque limiter (11) according to this invention can be sterilized by autoclaving without affecting the operation of the device.

The torque limiter (11) according to this invention is in particular suitable for surgical use.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

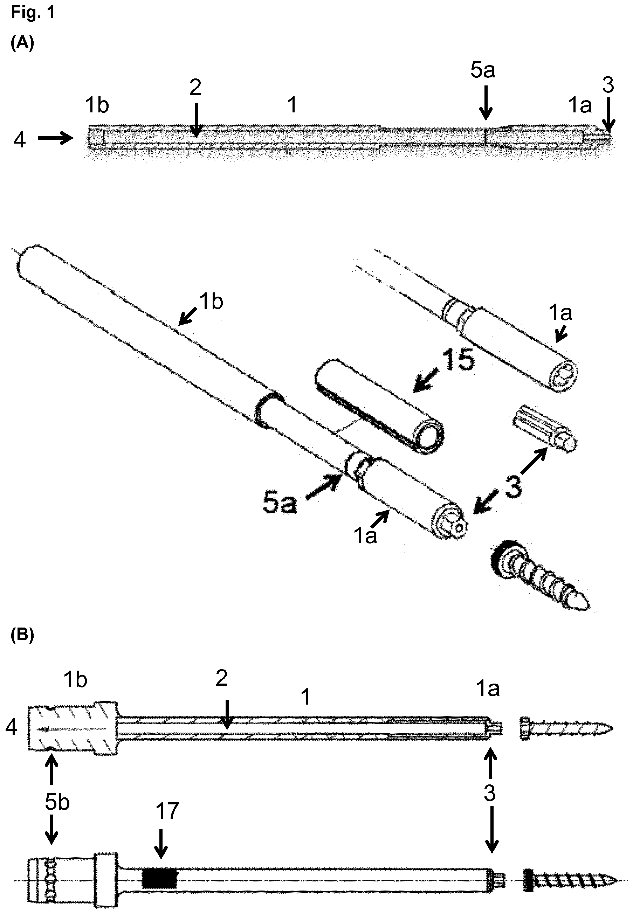

**Fig. 1****:** Represents two embodiments of an uninstalled sleeve member (1) according the invention, wherein the built-in mechanism for torque limitation (5) is defined by one or more breakpoints (5a) in said sleeve member **(A),** or wherein the built-in mechanism for torque limitation (5) is defined by one or more detents (5b) in said sleeve member **(B).** Furthermore, (A) shows a possible representation of a collar (15) which grips around said sleeve member (1) as well as a possible representation wherein the hollow portion (2), and bolt-or screw-fitting socket (3) are made from different types of material, e.g. plastic and metal (stainless steel) respectively.

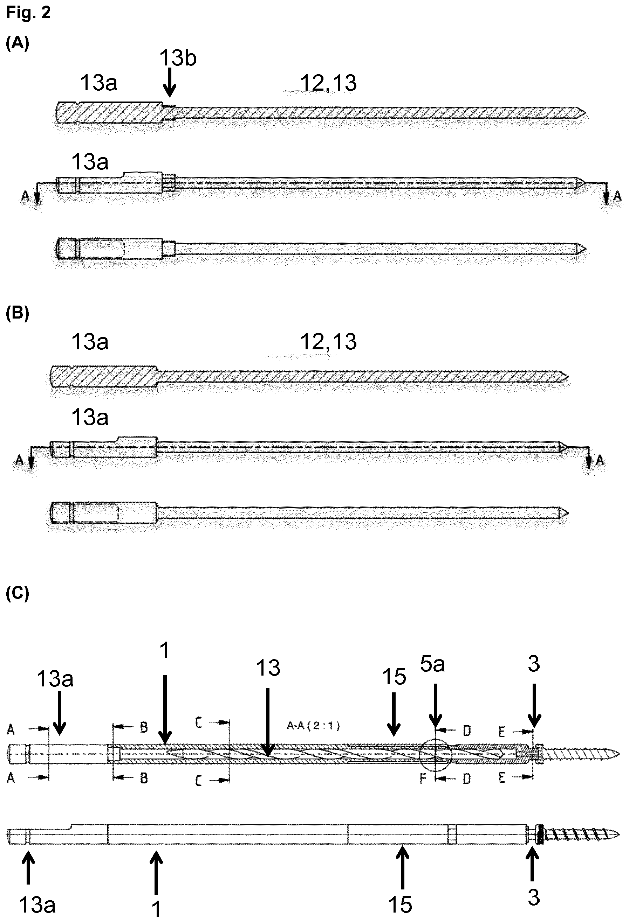

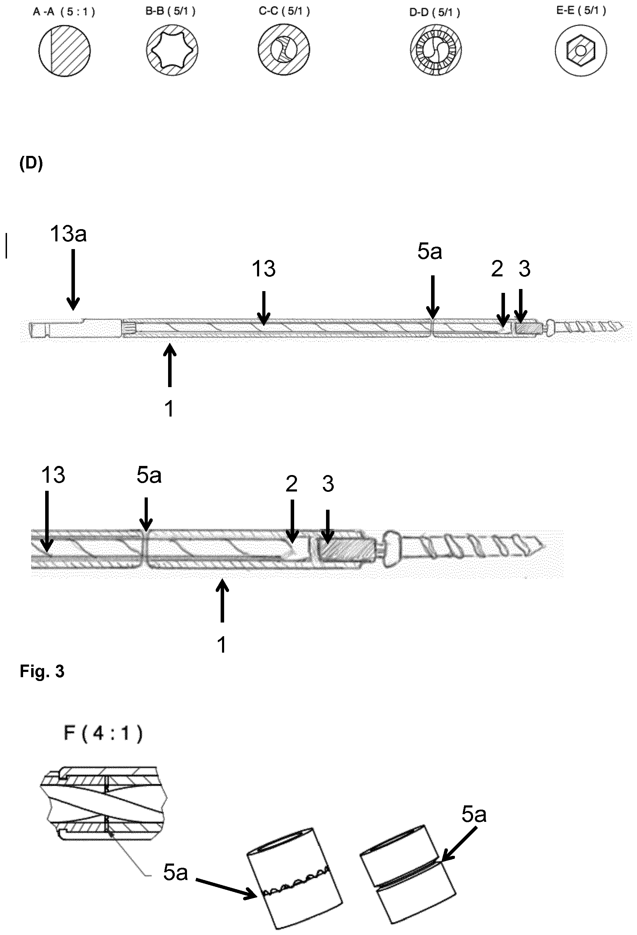

**Fig. 2****: (A and B)** Represent exemplary types of drills (13) suitable to be used in connection with the sleeve members (1) of the present invention, wherein the drill of **(A)** is further provided with a type of coupling (13b) for easy attachment to a torque limiter (11 or 19) according to this invention, and **(C)** an exemplary sleeve member (1) according to this invention, when installed on a suitable drill (13) and further (optionally) comprising a collar (15) around said sleeve member. (**D**) represents an exemplary sleeve member (1) according to this invention, when installed on a suitable drill (13) wherein the hollow portion (2), and bolt-or screw-fitting socket (3) are made from different types of material, e.g. plastic and metal (stainless steel) respectively.
**Fig. 3****:** Represents, two exemplary types of breakpoints (5a) suitable to be used in the sleeve member according to the present invention, and suitable to be used as the mechanism for torque limitation (5).

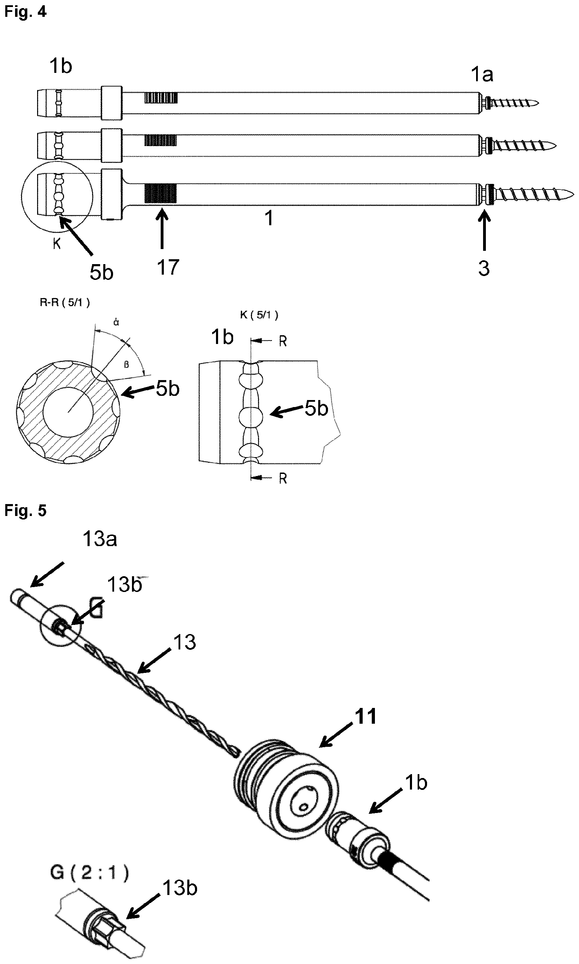

**Fig. 4****:** Represents a specific embodiment of the sleeve member (1) of the present invention, wherein the detents (5b) in the sleeve member (1) are adapted on the type/size of the bolt-or screw-fitting unit on the first end (1a) of said sleeve member. The detents can be asymmetrical to provide a different torque limit in both the clockwise and counter-clockwise direction.
**Fig. 5****:** Represents a suitable attachment means (13b) for releasable engaging the drill (13) to a torque limiter (11) according to this invention; and indicates the process of coupling the individual elements: drill (13), torque limiter (11) and hollow sleeve member (1).

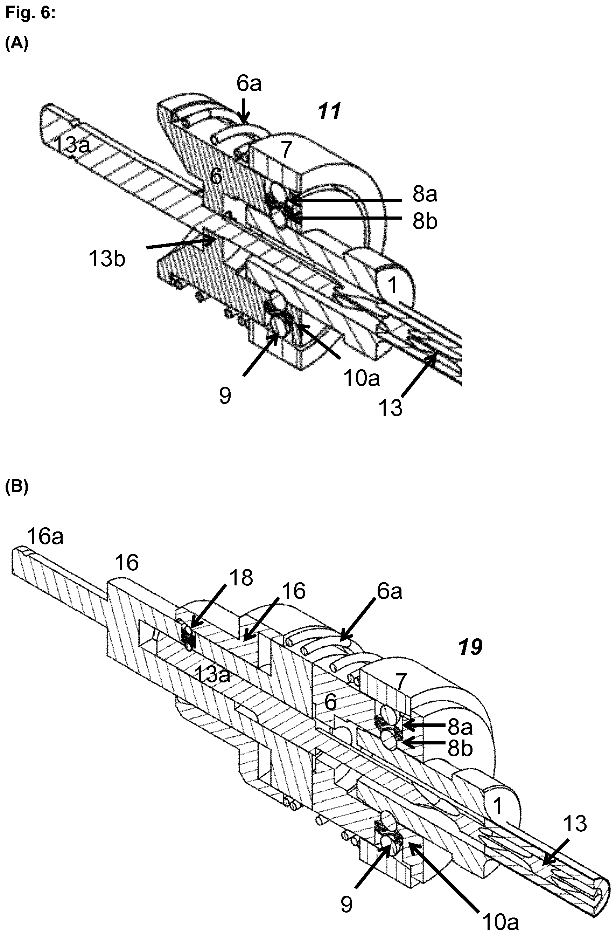

**Fig. 6****: (A)** Represents a first embodiment of a torque limiter (11) according to the present invention, more in particular used in the presence of a coupling system (attachment means 13b) present on the drill (13) or driven member (12), i.e. directly coupled to the drill or driven member; and **(B)** Represents a second embodiment of a torque limiter (19) according to the present invention, more in particular wherein the torque limiter itself comprises a release system for coupling to the drill (13) or driven member (12).

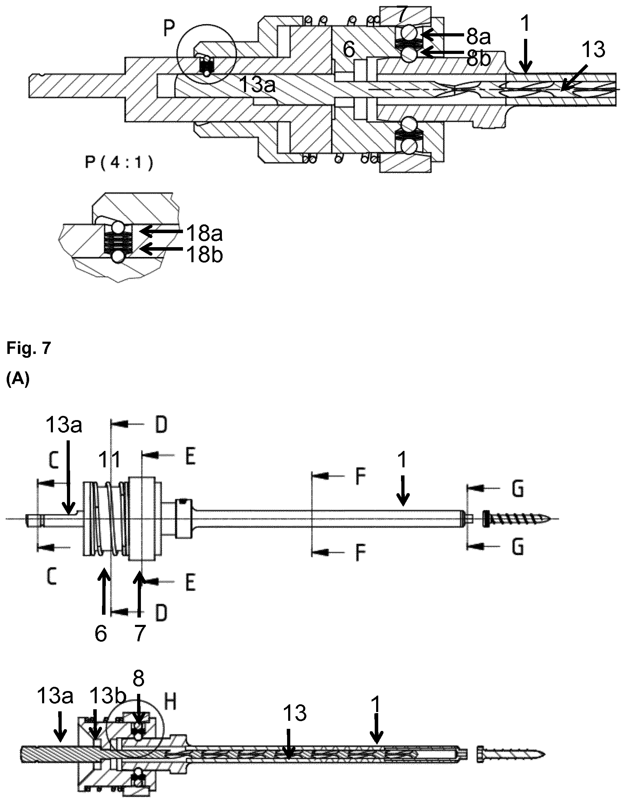

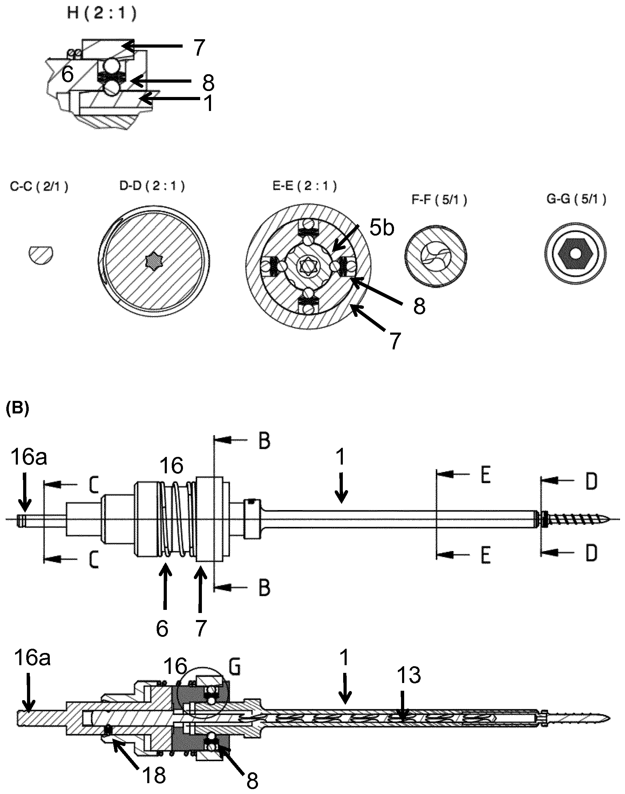

**Fig. 7****:** Represents the assembly of: **(A)** a torque limiter (11) with a drill (13) and hollow sleeve member (1) according to this invention, wherein the drill is provided with attachment means (13b) for releasably engaging the drill to the torque limiter; and **(B)** a torque limiter (11) with a drill (13) and hollow sleeve member (1) according to this invention, wherein the torque limiter is provided with a release system (18) for releasably engaging the drill to the torque limiter

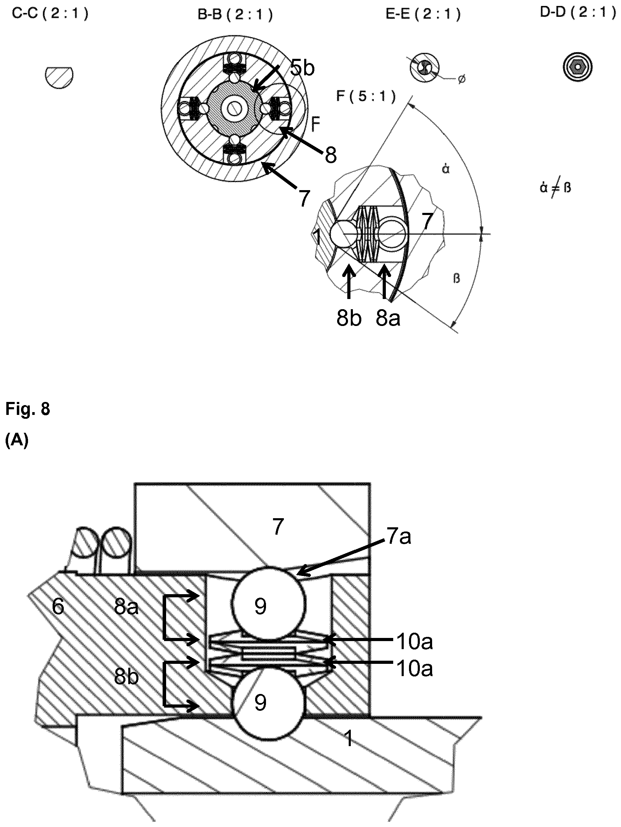

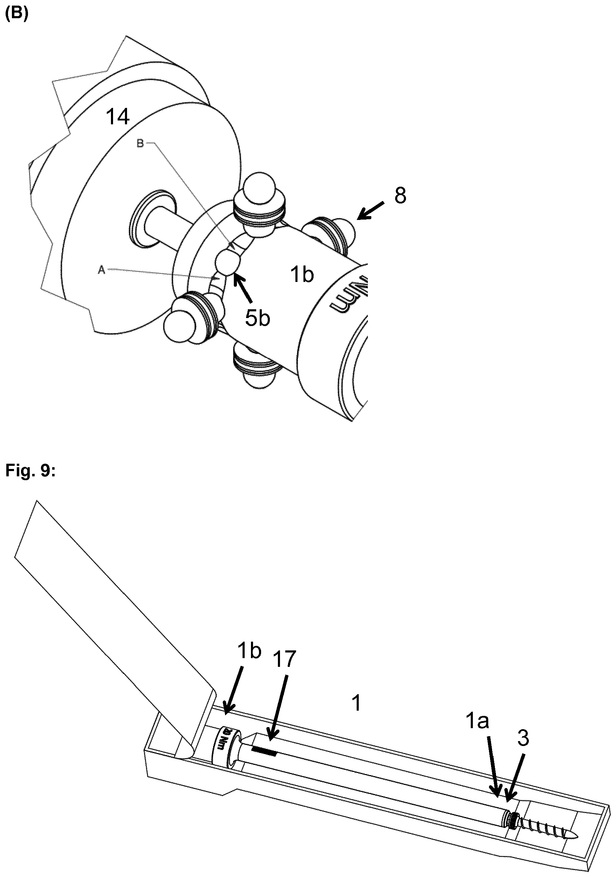

**Fig. 8****:** Represents **(A)** an embodiment of the elastic element (10) of the torque limiter, in particular of the double-transmission (8a, 8b) ball system, including spring washers (10a) and transmission balls (9), and **(B)** details of the association of the spring-rolling elements (8) of the torque limiter (11 or 19) of this invention with the detents (5b) in the second end (1b) of the sleeve member according to this invention.
**Fig. 9****:** Represents an embodiment of a kit comprising a sleeve member (1) and a screw or bolt, suitable to engage with the bolt-or screw fitting unit (3) at the first end (1a) of said sleeve member (1), optionally further comprising a barcode (17) or other means for easy identification of the torque limit and mounted screw or bolt.

### Numbering:

(1) Sleeve member
   (1a) First end of sleeve member
   (1b) Second end of sleeve member
(2) Hollow portion
(3) Bolt-or screw-fitting unit/socket
(4) Attachment means for releasably engaging sleeve member to drill or motor unit
(5) Built-in mechanism for torque limitation
   (5a) Breakpoints in sleeve member
   (5b) Detents or recesses in sleeve member
(6) First torque limiting element
   (6a) Spring element for displacing second torque limiting element
(7) Second torque limiting element
   (7a) Groove in second torque limiting element
(8) Spring-rolling elements
   (8a) First part of spring-rolling elements
   (8b) Second part of spring-rolling elements
(9) Transmission ball(s)
(10) Elastic element
   (10a) Spring Washers
(11) Torque limiter according to the first embodiment
(12) Driven member
   (12a) attachment means for releasable engaging the driven member to the motor unit.
   (12b) attachment means for releasable engaging the driven member to the torque limiter
(13) Drill
   (13a) attachment means for releasable engaging the drill to the motor unit
   (13b) attachment means for releasable engaging the drill to the torque limiter.
(14) Motor unit
(15) Collar
(16) Coupling system
   (16a) Coupling bit
   (16b) Locking System
(17) Identification code (e.g. barcode)
(18) Release System, such as for example spring-rolling elements
   (18a) First part of spring-rolling elements
   (18b) Second part of spring-rolling elements
(19) Torque limiter according to second embodiment

### DETAILED DESCRIPTION OF THE INVENTION

As indicated herein before, the present invention in particular aims at providing a system capable of quickly switching between drilling and screw-driving, while at the same time having a torque limiting system installed, which is adapted to the screw or bolt that is to be used in connection with the drilled hole.

Thereto, in a first aspect, the present invention provides a sleeve member (1) comprising:
- a hollow portion (2) adapted to embrace a drill (13),
- a bolt- or screw-fitting unit (3) on a first end (1a) of said sleeve member (1), and
- attachment means (4) on a second end (1b) of said sleeve member (1), for releasably engaging said sleeve member (1) to said drill (13) or to a motor unit (14);
said sleeve member (1) being characterized in that it comprises a built-in mechanism for torque limitation (5, in particular 5a and 5b) (see figures 1A and B).

By using this particular type of sleeve member (1), the operator can drill a hole using a motor unit fitted with a drill (13). Subsequently and without removing the drill, the operator can easily slide the hollow sleeve member (1) according to the invention over the drill (as for example illustrated in fig. 2C), and drive the same motor unit (14) to attach a bolt or screw capable to fit in the bolt-or screw-fitting unit (3) on the first end (1a) of said sleeve member. In addition, the operator should not worry about attaching a suitable torque limiter, or adjusting a pre-installed torque limiter, in accordance with the particular type/size of screw or bolt to be installed, since the sleeve member (1) according to this invention is already provided with a built-in mechanism for torque limitation (5). Hence, as evident, this type of sleeve member (1), significantly reduces the amount of time needed to switch from drilling to screw-driving with the correct torque limiter installed. This gain of time can in particular be crucial during an emergency surgical procedure.

It should be understood that the invention is not limited to motorized assemblies, and that the hollow sleeve member of the present invention, may also be used in association with non-motorized assemblies such as for example, classical screwdriver handles either or not with a quick coupling system, such as currently used in surgery and for example illustrated in WO2005120371.

In the context of the present invention, the terms 'screw' or 'bolt' are used to define a fastener capable of positioning objects, or holding objects together. For example, these may be used during osteosynthesis, wherein during a surgical procedure a connection must be made between 2 bones or bone fragments using plates, screws, bolts,...

The term bolt- or screw-fitting unit (3) as used herein, is defined as an ending on the sleeve member of the present invention, capable of receiving a bolt or screw, and adapted such that the driving action of a motor unit results in driving said bolt or screw into the object.

In the context of the present invention, the term 'motor unit' (14) refers to the driving means to which a drill bit and/or screwdriver bit can be fixed, and which is used for the automatic drilling or screwdriving action.

In the context of the present invention, the term 'drill bit' refers to the drill ending to be fitted on a motor unit and used to perforate an object such as for example a bone. Similar, a 'screwdriver bit' refers to the screwdriver ending to be fitted on the motor unit and used to drive a screw or bolt into a hole.

Evidently, each type/size of screw or bolt requires its own adapted torque limiter, thereby allowing the correct torque to be exerted on said particular type of screw or bolt. Hence, in a particular embodiment, the built-in mechanism for torque limitation (5) is adapted, depending on the type of bolt- or screw-fitting unit (3) in said sleeve member (1). In said embodiment, only screws or bolts that fit in/on the particular bolt- or screw-fitting unit (3) can be used in connection to a particular sleeve member (1), having the corresponding built-in mechanism for torque limitation (5) in place. This embodiment therefore significantly reduces or even removes the error margin of selecting a wrong torque limiter for a particular type of screw or bolt.

In a particular embodiment, said built-in mechanism for torque limitation (5) is defined by one or more breakpoints (5a) in said sleeve member (see figures 1A and 3). Any suitable means of providing such breakpoints between two or more parts of said sleeve member is possible, such as for example one or more shear pins, one or more weaker areas in the sleeve member, one or more holes in the sleeve member,... (see figure 3) While operating the sleeve member, the breakpoints will break and disconnect the two or more parts of said sleeve member, at a certain amount of torque asserted on said breakpoint. By using this type of torque limiting mechanism, a new sleeve member (1) comprising intact breakpoints needs to be used for each screw or bolt to be installed, since once the breakpoints (5a) have reached their maximum torque, and were broken, the sleeve member (1) can not be used again. Hence, this embodiment is particularly suitable for single-use applications, wherein a new sleeve member (1) is used for each new screw or bolt to be installed. Therefore, although the sleeve member may be made from any suitable material, such as for example metal (e.g. stainless steel), it is preferably made from a cheap (single-use) material such as a plastic material, for example polyethylene (PE), polypropylene (PP), polystyrene (PS), polyether ether ketone (PEEK) and the like. Alternatively, the sleeve member may be made from two or more different materials. For example, the hollow portion (2) may be made from a plastic material, while the bolt-or screw-fitting socket may be made from metal (e.g. stainless steel). This particular embodiment is further illustrated in figures 1A and 2D. Herein the hollow portion (2), and the built-in mechanism for torque limiting (5a) are made from a plastic material, whereas the bolt-or screw-fitting socket (3) is made from metal (e.g. stainless steel) and fits in the end of the sleeve member (1).

In this particular embodiment, the resistance of said breakpoints (5a) or pins is preferably adapted, depending on the type of bolt- or screw-fitting unit (3) in said sleeve member (1). Hence, as indicated above, only screws or bolts that fit in/on the particular bolt- or screw-fitting unit (3) can be used in connection to a particular sleeve member (1), wherein the resistance of the breakpoints (5a) is adapted based on said particular screws or bolts.

In yet a further embodiment, the sleeve member (1) according to the present invention, may comprise a collar (15), around a part of the sleeve member (1), by which the operator may hold the device in the correct position (see figure 1A). In addition, said collar (15) may provide protection for the environment against possible debris coming from breaking of the breakpoints of the built-in mechanism for torque limitation (5a). This is particularly relevant during surgery, wherein it is inacceptable that debris coming from the device would end up in the wound of the patient. When relevant, the collar may also serve the purpose of keeping both parts of the sleeve member together after breaking of the breakpoints. The collar (15) may be rotationally fixed to the sleeve member or may rotate around the sleeve member, in order for the operator to firmly hold the collar, while still allowing rotation of the sleeve member itself.

In yet another particular embodiment, the built-in mechanism for torque limitation (5) is defined by one or more detents or recesses (5b) in said sleeve member (1), preferably in the shaft of said sleeve member (see figure 1B). These detents (5b) are adapted to receive one or more spring-rolling elements (8) from a torque limiting device mounted on said drill (13) or motor unit (14) (see figure 8B). In this particular embodiment, the attachment means (4) are fully integrated with and correspond to the built-in mechanism for torque limitation (5) of the sleeve member (1). In this embodiment the sleeve member (1) may be made from any suitable material such as for example a plastic material, such as polyethylene (PE), polypropylene (PP), polystyrene (PS), polyether ether ketone (PEEK)...; or from metal such as stainless steel. Alternatively, the sleeve member may be made from two or more different materials. For example, the hollow portion (2) may be made from a plastic material, while the bolt-or screw-fitting socket may be made from metal (e.g. stainless steel).

In a particular embodiment, the size and/or form of said detents or recesses (5b) may be adjusted depending on the type of bolt- or screw-fitting unit (3) in said sleeve member (1) and are thus in fact adjusted on the type/size of the bolt or screw to be installed. This is for example illustrated in figure 4. In said instance, each type of screw or bolt has its own adapted sleeve member (1) with appropriately adapted detents (5b). Hence, the torque limiting device mounted on a drill (13) or motor unit (14) and comprising spring-rolling elements (8) for engaging with said detents, may be kept standard amongst different types of screws or bolts, whereas the adjustment in relation to the type of screw or bolt is achieved by the adapted detents (5b) in the specific sleeve members (1). This is in contrast to conventional devices, wherein the torque limiting device needs to be adjusted or interchanged for each type of screw or bolts to be installed. Hence, this embodiment allows the use of a single torque limiting device in association with a set of different screws and bolts each necessitating a specifically adjusted torque limiter.

In a particular embodiment, the detents (5b) in said sleeve member (1) may be symmetric in size or shape thereby allowing to apply a similar torque limitation in clockwise or counterclockwise direction. Alternatively the detents (5b) in said sleeve member (1) may be asymmetric in size or shape thereby allowing to apply a different torque limitation in clockwise or counterclockwise direction. Preferably, the detents (5b) are asymmetric in the rotational direction (i.e. bi-directional), thereby allowing a different maximum amount of torque in the clockwise or counterclockwise direction (see figure 4 and 7B, i.e. α≠β). In said instance, the detents (5b) are designed such that the torque to be transmitted is dependent on the direction of rotation. For example, when rotating the hollow sleeve (1) in the clockwise direction, i.e. fastening direction, the maximum amount of torque to be allowed (i.e. torque tolerance) on the device is preferably smaller in comparison with rotating the hollow sleeve in the counter-clockwise direction, i.e. unfastening direction. As such, although the maximum amount of torque may be reached in the clockwise direction, and further tightening the screw or bolt is impossible, it still remains possible to untighten the screw or bolt due to the higher torque tolerance in the counter-clockwise direction.

In a further aspect, the present invention provides a kit comprising a sleeve member (1) according to this invention, and one or more screws or bolts adapted to fit said bolt- or screw-fitting unit (3) on said first end (1a) of said sleeve member, such as for example illustrated in figure 9. Hence, in said embodiment, the sleeve member and screws/bolts are packed together such that at all instances, the correct screws/bolts are associated with the corresponding bolt- or screw-fitting socket/unit (3) and correct built-in system for torque limitation (5). This embodiment thus provides a particularly suitable combination for use during surgery, and significantly reduces the error-margin associated with the incorrect combination of screws/bolts and possible non-calibrated torque limiting systems.

The advantages of this particular embodiment during a surgical procedure become particularly evident when comparing the original process of changing between drill and screwdriver, with the new process as provided herein:

| **Original Process** | **Process of Invention** |
|---|---|
| Place drill on motor unit | Place drill on motor unit |
| Drill hole in bone/plate | Drill hole in bone/plate |
| Measure depth of hole | Measure depth of hole |
| Take screw/bolt out of sterile box | Assembling correct screw-hollow sleeve member combination, or receive the same from assistants/nurses, in sterile condition Take sleeve member of the invention with |
| Measure length of screw/bolt | |
| Remove drill bit from motor unit | |
| Attach correct torque limiter on motor unit | |
| Place screwdriver bit on torque limiter | |
| Place screw on screwdriver bit | Slide sleeve member of the invention over drill |
| Introduce screw/bolt in bone | Introduce screw/bolt in bone |
| Remove screwdriver bit from motor unit | Slide screwdriver of drill |
| Remove torque limiter from motor unit | |

Hence by using the sleeve member (1) of the present invention, at least 5 steps of the original process can be skipped. Experiments have shown that with the current invention, a significant reduction in operation time during a surgical procedure can be achieved.

The use of such a kit according to the invention also allows traceability during a surgical procedure. For example, when using a unique identification code (17) in association with each package, it is possible to track the actually used screws/bolts during and after the operation, at all instances. Such code may for example be located on the package and/or be applied on the side of the hollow sleeve, such as for example on the collar, whenever present. As an exemplary code, a barcode (17) may be used, which can be scanned during a surgical procedure before or after the application of the associated screw or bolt. Alternatively a removable label, either or not comprising such barcode, may be used. Said removable label may then be removed from the package or hollow sleeve during surgery and included in the patient's medical notes. Furthermore, the package, sleeve member and/or parts thereof, may also be provided with a colour code, for easily identifying the type/size of screw or bolt, which may also be provided with a colour code, to be installed; and/or the torque to be applied (e.g. expressed in Nm).

To allow the quick changing between drilling and screw-driving while simultaneously providing a corresponding torque limiter for each type of screw or bolt, the inventors have further developed a novel type of torque limiter (11 or 19) particularly suitable in connection with the sleeve member (1) as described herein above.

Hence, in a further aspect, the present invention provides a torque limiter (11) according to claim 9.

As already indicated hereinabove, the torque limiter according to this invention, is capable of embracing any suitable type of driven member (12), such as a screwdriver, drill, ... In the context of the present invention, the driven member is preferably a drill (13).

This type of torque limiter is for example illustrated in figure 6A and B.

In a more specific embodiment, the present invention provides as a torque limiter (19) according to claim 10.

Regardless of the built-in mechanism for torque limitation (5), the connection between the torque limiter (11 or 19) and driven member (12), drill (13) or motor unit (14) may be made by any suitable means, in as long as the torque limiter (11 or 16) is attached with the required rotational fixation to the driven member (12), drill (13) or motor unit (14). For example, the drill (13) may comprise attachment means (13b) having a specific shape and/or form (13a), capable of accommodating the torque limiter (11 or 19), and preventing the first torque limiting element (6) from rotating around its longitudinal axis (see figure 5 and 6A). Such attachment means (13b) may for example be represented by a star-coupling capable of engaging with a corresponding part of the torque limiter. Furthermore, the drill bit (13a) is preferably asymmetric, such that it can only fit in 1 direction in the motor unit (14). In a specific embodiment, said asymmetric ending may be duplicated in the shaft of the drill (13) or driven member (12) to embrace the first torque limiting element (6), and hence serving the purpose of attachment means (13b). As such, also said first torque limiting element (6) may only fit in 1 direction around the drill (13) or driven member (12), and at the same time provides for the required rotational fixation of the torque limiter (11).

Alternatively, the torque limiter may be coupled to the drill (13), or driven member (12) by using a further coupling system (16) as part of the torque limiter (19) as illustrated in Fig. 6B. Hence, in a particular embodiment, the torque limiter (19) according to this invention further comprises a coupling system (16) including a coupling bit (16a) for coupling said torque limiter to a motor unit; and a locking system (16b) for releasably engaging a drill (13) or driven member (12) to said torque limiter (19), by means of a release system (18).

In said embodiment, the torque limiter (19) according to the present invention, may be connected to the driven member (12), in particular a drill (13), by means of a coupling system (16) preferably as part of the torque limiter, as illustrated in Fig. 6b. Such coupling system (16) preferably comprises a coupling bit (16a) for installing it on the motor unit and is adapted to embrace the driven member (12) or drill (13). In this embodiment, the first torque limiting element (6) may be part of, or attached to the coupling system (16). The drill (13) or driven member (12) may be engaged to the coupling system by means of a release system (18), such as for example one or more further spring-rolling elements.

Regardless of the type of torque limiter (11 or 19), the hollow sleeve member of the invention (1) may be installed on the torque limiter (11 or 19) by means of the spring-rolling elements (8) in association with the second torque limiting element (7).

However, the torque limiter (19) with built-in coupling system (16), has further advantages in that it allows standard drills (13) or driven members (12) to be used in connection with said torque limiter, and does not require adaptation of existing driven members (12) or drills (13)

In a particular embodiment, the present invention provides a torque limiter (11 or 19) as defined herein above, wherein said second torque limiter element (7) comprises one or more grooves (7a) adapted to receive a first part (8a) of said one or more spring-rolling elements (8) (see figure 8A). Alternatively, said grooves may be replaced by any suitable means for accommodating the spring-rolling elements, such as for example, detents, recesses, trenches,...

In a further embodiment, the present invention provides the combination of a torque limiter (11) according to this invention, with a sleeve member (1) comprising a hollow portion (2) to embrace a driven member (12) or drill (13). Said torque limiter is adapted to releasably engage said second torque limiter element (7) to said driven member (12) or drill (13) by means of one or more spring-rolling elements (8) in the torque limiter, wherein the built-in mechanism for torque limitation (5) is present on said sleeve member (1) (see figure 7A). Said second torque limiter element (7) may be laterally displaced over the first torque limiting element (6) by means of a spring element (6a) wrapped around said first torque limiting element (6).

In said particular embodiment, said sleeve member preferably comprises one or more detents (5b) adapted to receive a second part of said spring-rolling elements (8b). More in particular, said sleeve is preferably a sleeve member (1) according to this invention. Hence, in this embodiment, the hollow sleeve (1) is attached to the drill (13) by means of this particular type of torque limiter (11) as illustrated in figure 7A. Hence, within this embodiment the attachment means (4) of the sleeve member are integrated with and consist of the built-in mechanism for torque limitation (5).

In yet a further embodiment, said one or more spring-rolling elements (8) each comprise 2 transmission balls (9) connected by an elastic element (10) such as a coil spring of spring washers, as illustrated in figure 8A. Preferably, spring washers (10a) are used, since the elasticity thereof changes very little after initial exposure to high temperatures required for autoclaving.

The force of the elastic elements (10) against the second torque limiting element (7) on the one hand and the driven member (12)/drill (13) (directly) or sleeve member (1) (indirectly) on the other hand, maintains the transmission balls (9) in the groove (7a) of said second torque limiting element (7); or in the detents of said driven member (12)/drill (13) or sleeve member (1) respectively. As the screw or bolt tightens in the object, it exerts a counter-torque on the sleeve member or driven member, which will be transmitted to the transmission balls (9). Once the counter-torque on the transmission balls exceeds the maximum pressure from the elastic element, the counter-torque will cause the transmission balls (9) to disengage from the detents or groove, thus preventing over-tightening of the screw or bolt.

The maximum amount of torque that may be exerted on the screw or bolt is a function of the force exerted by the elastic elements (10) against the transmission balls (9) and is defined by the depth, size and/or shape of the detents in the shaft of the driven member (12)/drill (13) and/or the detents (5b) in the shaft of the sleeve member (1). Thus the maximum amount of torque exerted on the screw or bolt can be adjusted by varying the depth, size and/or shape of the detents in the shaft of the driven member (12)/drill (13) and/or the detents (5b) in the shaft of the sleeve member (1). Hence a single type of torque limiter can be used in association with a set of different driven members (12) or sleeve members (1) each having adjusted detents dependent on the type or size of screw/bolt to be installed. Alternatively the depth, size and/or shape of the groove (7a) in the second torque limiting element (7) may be altered to adjust the maximum amount of torque to be exerted on the screw or bolt, however, in said instance, a separate torque limiter or a separate second torque limiting element according to the invention needs to be used in association with different types of screws or bolts.

In a further embodiment, the driven member (12) may be a drill (13) or a screwdriver, preferably a drill, more in particular a drill which may be embraced by a hollow sleeve, which serves the purpose of a screwdriving element such as a sleeve member (1) according to the present invention.

In yet a further embodiment, all components of the torque limiter (11) according to this invention can be sterilized by autoclaving without affecting the operation of the device. Hence, all of the components of the torque limiter (11 or 19) according to the invention are preferably constructed of stainless steel or other material that will reduce wear and abrasion, can be sterilized and can endure repeated autoclaving.

## Claims

1. A sleeve member (1) comprising:
- a hollow portion (2) adapted to embrace a drill (13),
- a bolt- or screw-fitting unit (3) on a first end (1a) of said sleeve member (1), and
- attachment means (4) on a second end (1b) of said sleeve member (1), for releasably engaging said sleeve member (1) to said drill (13) or to a motor unit (14);
said sleeve member (1) being **characterized in that** it comprises a built-in mechanism for torque limitation (5).

2. A sleeve member (1) according to claim 1, wherein said built-in mechanism for torque limitation (5) is defined by one or more breakpoints (5a) in said sleeve member (1).

3. A sleeve member (1) according to claim 2, wherein the resistance of said breakpoints (5a) is adapted, depending on the type of bolt- or screw-fitting unit (3) in said sleeve member (1).

4. A sleeve member (1) according to claim 1, wherein the attachment means (4) are fully integrated in, or correspond with, the built-in mechanism for torque limitation (5).

5. A sleeve member (1) according to claim 4, wherein said built-in mechanism for torque limitation (5) is defined by one or more detents (5b) in said sleeve member (1), said detents (5b) being adapted to receive one or more spring-rolling elements (8) from a torque limiter (11 or 16) mounted on said drill (13) or motor unit (14).

6. A sleeve member (1) according to claim 5, wherein said one or more detents (5b) are asymmetric thereby allowing to apply a different torque limitation in clockwise or counter-clockwise direction.

7. Use of a sleeve member (1) according to any one of claims 1 to 6 for surgical applications.

8. A kit comprising a sleeve member (1) according to any one of claims 1 to 6, and one or more bolts or screws adapted to fit said bolt- or screw-fitting unit (3).

9. A torque limiter (11) comprising:
- a first torque limiter element (6) capable of embracing at least a first part of a driven member (12), or drill (13);
- a second torque limiter element (7) for releasably engaging said first torque limiter element (6) with said driven member (12) or drill (13); and
- one or more spring-rolling elements (8) for releasably engaging said driven member (12) or drill (13) with said second torque limiter element by means of a built-in mechanism for torque limitation (5b) present on said driven member (12) or drill (13) or on a sleeve member (1) according to any one of claims 1, 4, 5 or 6 capable of embracing at least a second part of said driven member (12) or drill (13).

10. A torque limiter (19) comprising:
- a first torque limiter element (6) capable of embracing at least a first part of a driven member (12) or drill (13);
- a second torque limiter element (7) for releasably engaging said first torque limiter element (6) with said driven member (12) or drill (13);
- one or more spring-rolling elements (8) for releasably engaging said driven member (12) or drill (13) with said second torque limiter element by means of a built-in mechanism for torque limitation (5b) present on said driven member (12) or drill (13), or on a sleeve member (1) according to any one of claims 1, 4, 5 or 6 capable of embracing at least a second part of said driven member (12) or drill (13);
- a coupling system (16) including a coupling bit (16a) for coupling said torque limiter to a motor unit; and
- a locking system (16b) for releasably engaging said driven member (12) or drill (13) to said torque limiter (19), by means of a release system (18).

11. A torque limiter (11 or 19) according to any one of claims 9 and 10, wherein said second torque limiter element (7) comprises a groove (7a) adapted to receive a first part (8a) of said one or more spring-rolling elements (8).

12. A torque limiter (11 or 19) according to anyone of claims 9-11, wherein the built-in mechanism for torque limitation (5) comprises one or more detents (5b) adapted to receive a second part of said spring-rolling elements (8b).

13. A torque limiter (11 or 19) according to any one of claims 9-12, wherein each of said spring-rolling elements (8) comprises 2 transmission balls (9) connected by an elastic element (10), such as for example one or more spring washers (10a) or coil spring.

14. Use of a torque limiter (11) according to any one of claims 9-12 for surgical applications.

15. A combination of a torque limiter (11) according to any one of claims 9-12, with a sleeve member (1) as defined in any one of claims 1, 4, 5 and 6.

## Patentansprüche

1. Ein Hülsenelement (1), umfassend:
- einen hohlen Abschnitt (2) zum Einspannen eines Bohrers (13),
- ein Bolzen- oder Schraubenbefestigungsmodul (3) am einem ersten Ende (1a) des besagten Hülsenelements (1) und
- Befestigungsmittel (4) an einem zweiten Ende (1b) des besagten Hülsenelements (1) zur lösbaren Befestigung des besagten Hülsenelements (1) an besagtem Bohrer (13) oder einem Motormodul (14);
wobei besagtes Hülsenelement (1) **dadurch gekennzeichnet ist, dass** es einen eingebauten Mechanismus zur Drehmomentbegrenzung (5) umfasst.

2. Ein Hülsenelement (1) nach Anspruch 1, wobei besagter eingebauter Mechanismus zur Drehmomentbegrenzung (5) durch eine oder mehrere Haltemarken (5a) in besagtem Hülsenelement (1) definiert ist.

3. Ein Hülsenelement (1) nach Anspruch 2, wobei der Widerstand besagter Haltemarken (5a) jeweils in Abhängigkeit von der Art des Bolzen- oder Schraubenbefestigungsmoduls (3) in besagtem Hülsenelement (1) angepasst wird.

4. Ein Hülsenelement (1) nach Anspruch 1, wobei die Befestigungsmittel (4) vollständig in den eingebauten Mechanismus zur Drehmomentbegrenzung (5) integriert sind oder diesen bilden.

5. Ein Hülsenelement (1) nach Anspruch 4, wobei der besagte eingebaute Mechanismus zur Drehmomentbegrenzung (5) durch eine oder mehrere Feststellvorrichtungen (5b) in besagtem Hülsenelement (1) definiert ist und besagte Feststellvorrichtungen (5b) zur Aufnahme eines oder mehrerer Federrollenelemente (8) von einem Drehmomentbegrenzer (11 oder 16), der an besagtem Bohrer (13) oder Motormodul (14) montiert ist, geeignet sind.

6. Ein Hülsenelement (1) nach Anspruch 5, wobei besagte eine oder mehrere Feststellvorrichtungen (5b) asymmetrisch sind, um so die Anwendung unterschiedlicher Drehmomentbegrenzungen im oder gegen den Uhrzeigersinn zu ermöglichen.

7. Der Einsatz eines Hülsenelements (1) nach einem der Ansprüche 1 bis 6 für chirurgische Anwendungen.

8. Ein Kit, umfassend ein Hülsenelement (1) nach einem der Ansprüche 1 bis 6 und eine(n) oder mehrere Bolzen oder Schrauben, die für besagtes Bolzen- oder Schraubenbefestigungsmodul (3) angepasst sind.

9. Ein Drehmomentbegrenzer (11), umfassend:
- ein erstes Drehmomentbegrenzungselement (6), das mindestens einen ersten Teil eines angetriebenen Elements (12) oder Bohrers (13) einspannen kann;
- ein zweites Drehmomentbegrenzungselement (7) für eine lösbare Verbindung des besagten ersten Drehmomentbegrenzungselements (6) mit dem besagten angetriebenen Element (12) oder Bohrer (13); und
- ein oder mehrere Federrollenelemente (8) für eine lösbare Verbindung des besagten angetriebenen Elements (12) oder Bohrers (13) mit besagtem zweiten Drehmomentbegrenzungselement mittels eines eingebauten Mechanismus zur Drehmomentbegrenzung (5b), der an besagtem angetriebenen Element (12) oder Bohrer (13) oder an einem Hülsenelement (1) nach einem der Ansprüche 1, 4, 5 oder 6 vorhanden ist und mindestens einen zweiten Teil von besagtem angetriebenen Element (12) oder Bohrer (13) einspannen kann.

10. Ein Drehmomentbegrenzer (19), umfassend:
- ein erstes Drehmomentbegrenzungselement (6), das mindestens einen ersten Teil eines angetriebenen Elements (12) oder Bohrers (13) einspannen kann;
- ein zweites Drehmomentbegrenzungselement (7) für eine lösbare Verbindung des besagten ersten Drehmomentbegrenzungselements (6) mit dem besagten angetriebenen Element (12) oder Bohrer (13);
- ein oder mehrere Federrollenelemente (8) für eine lösbare Verbindung des besagten angetriebenen Elements (12) oder Bohrers (13) mit besagtem zweiten Drehmomentbegrenzungselement mittels eines eingebauten Mechanismus zur Drehmomentbegrenzung (5b), der an besagtem angetriebenen Element (12) oder Bohrer (13) oder an einem Hülsenelement (1) nach einem der Ansprüche 1, 4, 5 oder 6 vorhanden ist und mindestens einen zweiten Teil von besagtem angetriebenen Element (12) oder Bohrer (13) einspannen kann;
- ein Kupplungssystem (16) einschließlich eines Kupplungsstücks (16a) zur Kopplung besagten Drehmomentbegrenzers mit einem Motormodul; und
- ein Verriegelungssystem (16b) für die lösbare Verbindung von besagtem angetriebenen Element (12) oder Bohrer (13) mit besagtem Drehmomentbegrenzer (19) mittels eines Auslösesystems (18).

11. Ein Drehmomentbegrenzer (11 oder 19) nach einem der Ansprüche 9 und 10, wobei das besagte zweite Drehmomentbegrenzungselement (7) eine Nut (7a) umfasst, die zur Aufnahme eines ersten Teils (8a) von besagten einem oder mehreren Federrollenelementen (8) geeignet ist.

12. Ein Drehmomentbegrenzer (11 oder 19) nach einem der Ansprüche 9 bis 11, wobei der eingebaute Mechanismus zur Drehmomentbegrenzung (5) eine oder mehrere Feststellvorrichtungen (5b) umfasst, die zur Aufnahme eines zweiten Teils von besagten Federrollenelementen (8b) geeignet sind.

13. Ein Drehmomentbegrenzer (11 oder 19) nach einem der Ansprüche 9 bis 12, wobei jedes der besagten Federrollenelemente (8) 2 Übertragungskugeln (9) umfasst, die durch ein elastisches Element (10) wie beispielsweise eine oder mehrere Federscheiben (10a) oder eine Schraubenfeder verbunden sind.

14. Der Einsatz eines Drehmomentbegrenzers (11) nach einem der Ansprüche 9 bis 12 für chirurgische Anwendungen.

15. Eine Kombination eines Drehmomentbegrenzers (11) nach einem der Ansprüche 9 bis 12 mit einem Hülsenelement (1) nach einem der Ansprüche 1, 4, 5 und 6.

## Revendications

1. Élément manchon (1) comprenant :
- une partie creuse (2) adaptée pour enserrer une mèche (13),
- une unité adaptable boulon/vis (3) à la première extrémité (1a) dudit élément manchon (1), et
- un moyen de fixation (4) à la deuxième extrémité (1b) dudit élément manchon (1), permettant de solidariser, avec possibilité de désolidarisation, ledit élément manchon (1) avec ladite mèche (13) ou une unité à moteur (14),
ledit élément manchon (1) **se caractérisant par le fait qu'**il comporte un mécanisme intégré destiné à la limitation de couple (5).

2. Élément manchon (1) selon la revendication 1, dans lequel ledit mécanisme intégré destiné à la limitation de couple (5) est défini par un ou plusieurs point(s) de rupture (5a) dans ledit élément manchon (1).

3. Élément manchon (1) selon la revendication 2, dans lequel la résistance desdits points de rupture (5a) est adaptée en fonction du type d'unité adaptable boulon/vis (3) dans ledit élément manchon (1).

4. Élément manchon (1) selon la revendication 1, dans lequel le moyen de fixation (4) est complètement incorporé dans, ou en correspondance avec, le mécanisme intégré destiné à la limitation de couple (5).

5. Élément manchon (1) selon la revendication 4, dans lequel ledit mécanisme intégré destiné à la limitation de couple (5) est défini par un ou plusieurs encliquetage(s) (5b) dans ledit élément manchon (1), lesdits encliquetages (5b) étant adaptés pour recevoir une ou plusieurs pièce(s) mobile(s) sur ressort (8) provenant d'un limiteur de couple (11 ou 16) monté sur ladite mèche (13) ou unité à moteur (14).

6. Élément manchon (1) selon la revendication 5, dans lequel le(s)dit(s) encliquetage(s) (5b) sont asymétriques et permettent ainsi d'appliquer une limitation de couple différente dans le sens horaire et dans le sens antihoraire.

7. Utilisation d'un élément manchon (1) selon l'une quelconque des revendications 1 à 6 dans le cadre d'applications chirurgicales.

8. Kit comprenant un élément manchon (1) selon l'une quelconque des revendications 1 à 6, et un(e) ou plusieurs boulon(s) ou vis prévu(e)(s) pour être engagé(e)(s) dans l'unité adaptable boulon/vis (3).

9. Limiteur de couple (11) comprenant :
- un premier élément de limiteur de couple (6) capable de recevoir au moins une première partie d'un élément entraîné (12), ou d'une mèche (13) ;
- un deuxième élément de limiteur de couple (7) permettant de solidariser, avec possibilité de désolidarisation, ledit premier élément de limiteur de couple (6) avec ledit élément entraîné (12) ou ladite mèche (13) ; et
- une ou plusieurs pièce(s) mobile(s) sur ressort (8) permettant de solidariser, avec possibilité de désolidarisation, ledit élément entraîné (12) ou ladite mèche (13) avec ledit deuxième élément de limiteur de couple au moyen d'un mécanisme intégré destiné à la limitation de couple (5b) présent sur ledit élément entraîné (12) ou ladite mèche (13), ou sur un élément manchon (1) selon l'une quelconque des revendications 1, 4, 5 ou 6 capable de recevoir au moins une deuxième partie dudit élément entraîné (12) ou de ladite mèche (13).

10. Limiteur de couple (19) comprenant :
- un premier élément de limiteur de couple (6) capable de recevoir au moins une première partie d'un élément entraîné (12) ou d'une mèche (13) ;
- un deuxième élément de limiteur de couple (7) permettant de solidariser, avec possibilité de désolidarisation, ledit premier élément de limiteur de couple (6) avec ledit élément entraîné (12) ou ladite mèche (13) ;
- une ou plusieurs pièce(s) mobile(s) sur ressort (8) permettant de solidariser, avec possibilité de désolidarisation, ledit élément entraîné (12) ou ladite mèche (13) avec ledit deuxième élément de limiteur de couple au moyen d'un mécanisme intégré destiné à la limitation de couple (5b) présent sur ledit élément entraîné (12) ou ladite mèche (13) ou sur un élément manchon (1) selon l'une quelconque des revendications 1, 4, 5 et 6 capable de recevoir au moins une deuxième partie dudit élément entraîné (12) ou de ladite mèche (13) ;
- un système de connexion (16) comprenant une pièce de raccordement (16a) pour connecter ledit limiteur de couple à une unité à moteur ; et
- un système de verrouillage (16b) permettant de solidariser, avec possibilité de désolidarisation, ledit élément entraîné (12) ou ladite mèche (13) avec ledit limiteur de couple (19), au moyen d'un système de déblocage (18).

11. Limiteur de couple (11 ou 19) selon la revendication 9 ou 10, dans lequel ledit deuxième élément de limiteur de couple (7) comprend une gorge (7a) adaptée pour recevoir la première partie (8a) de ladite/desdites pièce(s) mobile(s) sur ressort (8).

12. Limiteur de couple (11 ou 19) selon l'une quelconque des revendications 9 à-11, dans lequel le mécanisme intégré destiné à la limitation de couple (5) comprend un ou plusieurs encliquetage(s) (5b) adaptés pour recevoir la deuxième partie desdites pièces mobiles sur ressort (8b).

13. Limiteur de couple (11 ou 19) selon l'une quelconque des revendications 9 à 12, dans lequel chacune desdites pièces mobiles sur ressort (8) comprend 2 billes de transmission (9) connectées par un élément élastique (10), pouvant être par exemple une ou plusieurs rondelle(s) élastique(s) (10a) ou un ressort hélicoïdal.

14. Utilisation d'un limiteur de couple (11) selon l'une quelconque des revendications 9 à 12 dans le cadre d'applications chirurgicales.

15. Combinaison d'un limiteur de couple (11) selon l'une quelconque des revendications 9 à 12, avec un élément manchon (1) tel que défini dans l'une quelconque des revendications 1, 4, 5 et 6.
